# EUROPÄISCHE PATENTSCHRIFT

(11) **EP 2 121 082 B1**
(45) Veröffentlichungstag und Bekanntmachung des Hinweises auf die Patenterteilung: **14.08.2019**
(21) Anmeldenummer: 08701035.1
(22) Anmeldetag: 09.01.2008
(51) Int. Cl.: A61M 5/30, A61M 5/24, A61M 5/31, A61M 5/20, A61M 5/315

(54) **EINWEGINJEKTOR MIT MINDESTENS EINEM ZUGHAKEN**
SINGLE-USE INJECTOR WITH AT LEAST ONE DRAW HOOK
INJECTEUR À UNE VOIE COMPRENANT AU MOINS UN CROCHET DE TRACTION

(30) Priorität: 27.01.2007 DE 102007004211
(43) Veröffentlichungstag der Anmeldung: 25.11.2009
(73) Patentinhaber: LTS LOHMANN Therapie-Systeme AG, 56626 Andernach (DE)
(72) Erfinder: ASMUSSEN, Bodo, 56170 Bendorf (DE); HOFFMANN, Hans-Rainer, 56566 Neuwied (DE); MATUSCH, Rudolf, 35041 Marburg (DE); WORTMANN, Uwe, 35037 Marburg (DE)
(74) Vertreter: Thämer, Wolfgang
(86) Internationale Anmeldenummer: PCT/EP2008/000102
(87) Internationale Veröffentlichungsnummer: WO 2008/089886

(56) Entgegenhaltungen:
- EP-A1- 0 595 508
- WO-A-03/092771
- WO-A-2007/002052
- DE-A1-102004 060 146

## Beschreibung

Die Erfindung betrifft einen nadelfreien Einweginjektor mit einem Gehäuse, in dem oder an dem - jeweils zumindest bereichsweise - mindestens ein mechanischer Federenergiespeicher angeordnet ist, mindestens eine -zumindest zeitweise wirkstoffbefüllbare - Zylinder-Kolben-Einheit befestigt angeordnet ist, mindestens ein Kolbenbetätigungsstempel angeordnet ist und mindestens eine Auslöseeinheit angeordnet ist, wobei der Federenergiespeicher mindestens ein vorgespanntes Federelement umfasst und wobei zumindest ein Teil des Kolbenbetätigungsstempels zwischen dem Federenergiespeicher und dem Kolben der Zylinder-Kolben-Einheit positioniert ist und wobei der federbelastete Kolbenbetätigungsstempel mindestens einen zumindest bereichsweise querbeweglichen Zugstab aufweist, der mittels eines Abstützabschnitts den gespannten Federenergiespeicher an mindestens einer Auflagefläche des Gehäuses abstützt.

Aus der EP 0 710 13 0 B1 ist u.a. ein derartiger Injektor bekannt. Er ist so konstruiert, dass sich die einzelnen Baugruppen Federenergiespeicher, Zylinderkolbeneinheit und Auslöseeinheit nicht voneinander trennen öder separat handeln lassen. Die Auslöseeinheit hat ein Rastgesperre, bei dem ein quer zur Injektormittellinie bewegter Schieber den Kolbenbetätigungsstempel über eine Kerbe oder eine Gewinderillung blockiert oder freigibt.

Aus der DE 10 2004 060 146 A1 ist ein Nadelinjektor mit einem zweiteiligen Gehäuse bekannt. Relativ zum Gehäuse ist eine Zylinder-Kolben-Einheit in Längsrichtung beweglich angeordnet. Weiterhin ist das Gehäuse relativ zu einer Führungshülse verschiebbar, an der sich vor dem Auslösen Rastnasen eines mit dem Kolben verbundenen Treibteils abstützen. Das Auslösen erfolgt durch Verschieben des Gehäuses relativ zum Treibteil und zum Kolben.

Die WO 03/092771 offenbart einen Nadelinjektor, in dem ebenfalls eine Zylinder-Kolben-Einheit in einem Gehäuse verschiebbar gelagert ist. Ein vom Kolben mittels eines Flüssigkeitspolster getrennter, federvorgespannter Betätigungskolben soll durch manuelles Schwenken eines Hebels ausgelöst werden. Hierbei wird zunächst die Zylinder-Kolben-Einheit verfahren, bevor ihr Kolben in die Ausstoßrichtung verfahren wird.

Eine weitere Ausführung einer Injektionsvorrichtung mit einer Nadel und mehreren korrespondierenden Federn ist aus der WO 2007/002052 A2 bekannt.

Die EP 0 595 508 A1 offenbart einen nadellosen Einweginjektor, bei dem der Kolben Teil des Kolbenbetätigungsstempels ist. Zur Freigabe der Zugstäbe wird ein Sperrstück in der Längsrichtung des Injektors eingeschoben. Die sich entspannende Feder zieht die Zugstäbe von der gehäuseseitigen Anlage.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruches gelöst. Dazu ist in der Auslöseeinheit mindestens ein Auslöseelement quer zur Mittellinie des Gehäuses längsverschieblich gelagert, das bei einer Betätigung ein Wegbewegen des Abstützabschnitts von der Auflagefläche bewirkt oder freigibt.

Mit der Erfindung wird ein nadelfreier Einmalinjektor vorgestellt, dessen Kolbenbetätigungsstempel bei einem Auslösevorgang des Einweginjektors eine Bewegung erfährt, die quer zur Längsrichtung und/oder quer zur Mittellinie des Einweginjektors orientiert ist. Dazu liegen zum Vorspannen und Halten des Federenergiespeichers ein oder mehrere Teile des Kolbenbetätigungsstempels mit mindestens einem Umgriff oder einem Haken am Gehäuse oder einem am Gehäuse angeordneten Bauteil an. Ggf. können auch nur bestimmte Teile oder Bereiche des Kolbenbetätigungsstempels gegenüber dem Gehäuse des Einweginjektors beweglich gestaltet sein. Zum Auslösen des Einweginjektors werden die Umgriffe oder Haken von ihrer gehäuseseitigen Auflagefläche heruntergeschoben, so dass sich der Kolbenbetätigungsstempel - unter der Wirkung des Federenergiespeichers - zumindest annähernd parallel zur Mittellinie des Einweginjektors bewegen kann.

Weitere Einzelheiten der Erfindung ergeben sich aus den Unteransprüchen und den nachfolgenden Beschreibungen von einigen schematisch dargestellten Ausführungsbeispielen.
- Figur 1:: Einweginjektor mit einem Zugstab;
- Figur 2:: wie Figur 1, jedoch entsichert und betätigt;
- Figur 3:: wie Figur 2, jedoch nach dem Medikamentenausstoß;
- Figur 4:: Einweginjektor mit mehreren Zugstäben;
- Figur 5:: wie Figur 4, jedoch entsichert und betätigt;
- Figur 6:: wie Figur 5, jedoch nach dem Medikamentenausstoß;
- Figur 7:: Draufsicht auf den Boden des Einweginjektors nach Figur 4 jedoch ohne die gestufte Auslösehülse;

- Figur 8:: Einweginjektor mit mehreren Zugstäben, die nach innen weisende Zughaken haben;
- Figur 9:: wie Figur 8, jedoch entsichert und betätigt;
- Figur 10:: wie Figur 9, jedoch nach dem Medikamentenausstoß;
- Figur 11:: Draufsicht auf das Sicherungselement des Einweginjektors nach Figur 8 jedoch ohne das Auslöseelement;

- Figur 12:: Einweginjektor mit mehreren nach außen federnden Zugstäben;
- Figur 13:: wie Figur 12, jedoch entsichert und betätigt;
- Figur 14:: wie Figur 13, jedoch nach dem Medikamentenausstoß;
- Figur 15:: Seitenansicht zum Einweginjektor nach Figur 12, jedoch vor der Benutzung;
- Figur 16:: Seitenansicht des Einweginjektors nach Figur 12, jedoch ohne Zylinderkolbeneinheit und Stützhülse;
- Figur 17:: Einweginjektor mit mehreren nach innen federnden Zugstäben;
- Figur 18:: wie Figur 17, jedoch entsichert und betätigt;
- Figur 19:: wie Figur 18, jedoch nach dem Medikamentenausstoß;
- Figur 20:: Seitenansicht zum Einweginjektor nach Figur 17, jedoch vor der Benutzung.

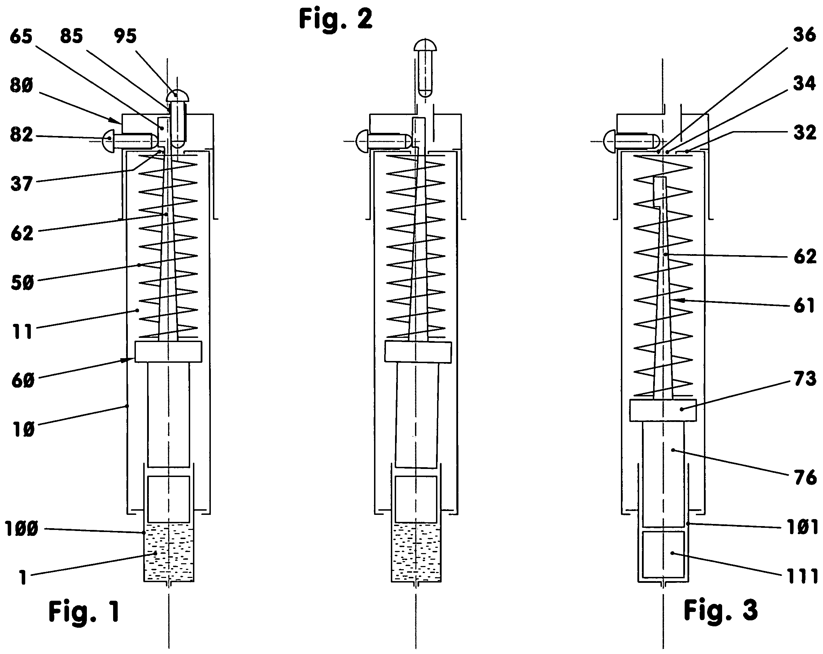

Die Figuren 1 bis 3 zeigen das vereinfachte Prinzip eines Einweginjektors mit einem dauergeladenen Federenergiespeicher. Der Einweginjektor besteht aus einem Gehäuse (10), einer z.B. befüllten Zylinder-Kolben-Einheit (100), einem Kolbenbetätigungsstempel (60) mit Zughaken (62) und einer Schraubendruckfeder (50) als Federspeicher. Zudem sitzt auf dem Gehäuse (10) eine Auslöseeinheit (80), in der ein Auslöseelement (82) und ein Sicherungselement (95) angeordnet sind.

Das Gehäuse ist ein topfförmiger, unten offener Hohlkörper mit obenliegendem Boden (32). Der Boden (32) hat z.B. eine außermittige Öffnung (34), durch die nach Figur 1 der Zughaken (62) hindurchgesteckt ist. Der Zughaken (62) liegt mit seinem Abstützabschnitt (65) auf der Auflagefläche (37) des Gehäuses (10) auf.

Der Kolbenbetätigungsstempel (60) ist in drei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der mittlere Bereich ist der Stempelteller (73). Der Stempelteller (73) ist eine flache, zumindest bereichsweise zylindrische Scheibe, deren Außendurchmesser einige Zehntel Millimeter kleiner ist als der Innendurchmesser des Gehäuses (10) im Mantelbereich (31). Der obere Bereich ist der Zughaken (62).

Im unteren Teil des Gehäuses (10) ist die Zylinder-Kolben-Einheit (100) befestigt. Die Zylinder-Kolben-Einheit (100) besteht hier aus einem mit einer Injektionslösung (1) befüllten Zylinder (101), in dem ein Kolben (111) in der hinteren Position sitzt. Oberhalb des Kolbens (111) ist im Gehäuse (10) der Kolbenbetätigungsstempel (60) z.B. so angeordnet, das er den Kolben zwar nicht berührt, jedoch mit seinem unteren Ende im oberen Bereich des Zylinders (101) seitlich geführt wird.

Zwischen dem Stempelteller (73) und dem obenliegenden Boden (32) des Gehäuses (10) sitzt vorgespannt die Schraubendruckfeder (50).

Auf dem Gehäuse (10) steckt die Auslöseeinheit (80). Letztere trägt in ihrer großteils geschlossenen Stirnwand eine Hülse (85), in der ein stiftförmiges Sicherungselement (95) steckt. Das Sicherungselement (95) ist in Kombination mit dem Gehäuse (10) so positioniert, dass es den Zughaken (62) an der Kante (36) der Öffnung (34) hält, an die sich die Auflagefläche (37) anschließt. Das Sicherungselement (95) verhindert im eingebauten Zustand ein unbeabsichtigtes Verschieben des Zughakens (62) quer zur Längsrichtung des Kolbenbetätigungsstempels (60).

Beispielsweise quer zur Mittellinie (5) des Gehäuses (10) ist in der Auslöseeinheit (80) das Auslöseelement (82) längsverschiebbar gelagert. Zum Betätigen des Einweginjektors wird nach Figur 2 das Sicherungselement (95) gezogen, der Einweginjektor gegenüber dem Patienten in Position gebracht und anschließend z.B. mit einem Finger der den Einweginjektor tragenden Hand das Auslöseelement (82) gedrückt. Das Auslöseelement (82) schiebt hierbei den Abstützabschnitt (65) - unter einer geringfügigen Schrägstellung des gesamten Kolbenbetätigungsstempels (60) - von der Abstützfläche (37). Die Schiebebewegung verläuft quer zur Längsachse bzw. Mittellinie (5) des Einweginjektors. In der Folge rutscht der Abstützabschnitt (65) unter der Wirkung der Schraubendruckfeder (50) durch die Öffnung (34) in das Innere (11) des Gehäuses (10). Hierbei wird die Zylinder-Kolben-Einheit (100) entleert, vgl. Figur 3.

Bei diesem Prinzip kann der Kolbenschieber (76) auch als separates Bauteil ausgeführt sein. Hierzu ist er dann an der Innenwandung des Gehäuses (10) geführt. Es ist auch möglich, den Kolbenschieber (76) als Kolbenstange am Kolben (111) anzuformen und dabei die Kolbenstange nur durch den Kolben (111) und/oder durch eine - z.B. bereichsweise - Anlage an der Innenwandung des Zylinders (101) zu führen. Selbstverständlich können sich der Kolbenschieber (76) und die Kolbenstange den Raum zwischen dem Stempelteller (73) und dem Kolben (111) beliebig aufteilen.

Die Art der Auslösung ist nicht auf die hier beschriebene Variante beschränkt. Anstelle des quer verschiebbaren Auslöseelements (82) kann z.B. u.a. ein Exzentergetriebe, ein Schraubgetriebe oder ein Hebelgetriebe treten.

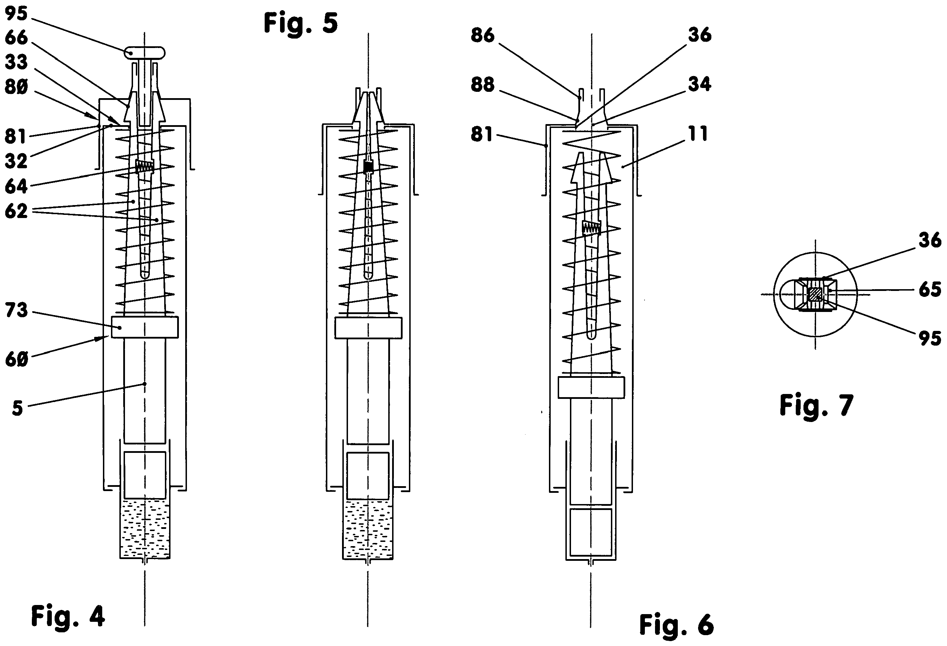

In den Figuren 4 bis 6 ist eine Verfeinerung des Prinzips nach den Figuren 1 bis 3 dargestellt. Der Kolbenbetätigungsstempel (60) weist oberhalb des Stempeltellers (73) z.B. zwei baugleiche Zughaken (62) auf. Beide Zughaken (62) liegen einander spiegelbildlich gegenüber. Sie bestehen beispielsweise aus einem federelastischen Material. Beide Zughaken (62) liegen Rücken an Rücken, wobei sie versuchen, sich blattfederartig auseinander zu drücken, so dass sie am Rand (36) der Öffnung (34) z.B. unter Vorspannung anliegen. Ihre Federrichtung wird symbolisiert durch eine ersatzweise zwischen ihnen querliegende Schraubendruckfeder (64).

Selbstverständlich kann eine derartige Schraubendruckfeder (64) auch real verwendet werden, sofern z.B. die Zughaken (62) am Stempelteller (73) mittels Schwenkgelenke angelenkt werden. Die Schwenkachsen dieser Schwenkgelenke lägen dann quer zur Gehäusemittellinie (5) und normal zur Darstellungsebene nach den Figuren 4 bis 6.

Die Abstützabschnitte (65), über die die Zughaken (62) an der z.B. ebenen Außenfläche (33) des Bodens (32) anliegen, haben bei dieser Variante eine keilförmige oder eine kegelstumpfmantelförmige Außenkontur (66). Hierbei verjüngt sich der Querschnitt der Abstützabschnitte (65) entlang der Mittellinie (5) nach oben hin. In den Figuren 4 bis 6 sind die Außenkonturen (66) Teile von Pyramidenflächen, die eine theoretische Pyramidenspitze haben, die oberhalb des Bodens (32) auf der Mittellinie (5) liegt, vgl. Figur 4, 5 und 7.

Zwischen den Enden der Zughaken (62) steckt ein stiftförmiges Sicherungselement (95), vgl. auch Figur 7. Hier hat es z.B. einen rechteckigen Querschnitt. Das Sicherungselement (95), das z.B. im Gehäuse (81) der Auslöseeinheit (80) gelagert ist, blockiert die Zughaken (62) mechanisch in ihrer Sperrstellung.

Das Auslösegehäuse (81) ist topfförmig ausgebildet und sitzt längsverschieblich auf dem hinteren Teil des Gehäuses (10). Auf dem Boden des Auslösegehäuses (81) ist z.B. ein Rechteckrohr (86) aufgesetzt, das zugleich das Sicherungselement (95) führt. Am Übergang vom Boden zum Rechteckrohr (86) weist letzteres abgeschrägte Bereiche (88) auf. Die abgeschrägten Bereiche (88) bilden die auslösegehäuseseitigen Keilflächen.

Nach einem Entfernen des Sicherungselements (95) kann das Auslösegehäuse (81) - wie ein Druckknopf - nach unten verschoben werden. Hierbei legen sich die auslösegehäuseseitigen Keilflächen (88) an den Zughaken (62) an und drücken diese - gegen die Wirkung der symbolisch dargestellten Druckfeder (64) - soweit zusammen, dass die Abstützabschnitte (65) durch die Öffnung (34) passen. Die Abstützabschnitte (65) und die auslösegehäuseseitigen Keilflächen (88) bilden dabei ein Schiebekeilgetriebe. Ggf. können die Keilflächen (88) und/oder die Flächen der keilförmigen Außenkontur (66) der Abstützabschnitte (65) ein- oder mehrachsig, z.B. zylindrisch oder sphärisch, gekrümmt sein, so dass eine gekrümmte Fläche an einer ebenen Fläche oder einer Fläche anderer Krümmung entlanggleitet.

Sobald die z.B. elastisch verformten Zughaken (62) als Teile des Kolbenbetätigungsstempels (60) das Innere (11) des Gehäuses (10) erreicht haben, federn sie z.B. wieder auseinander.

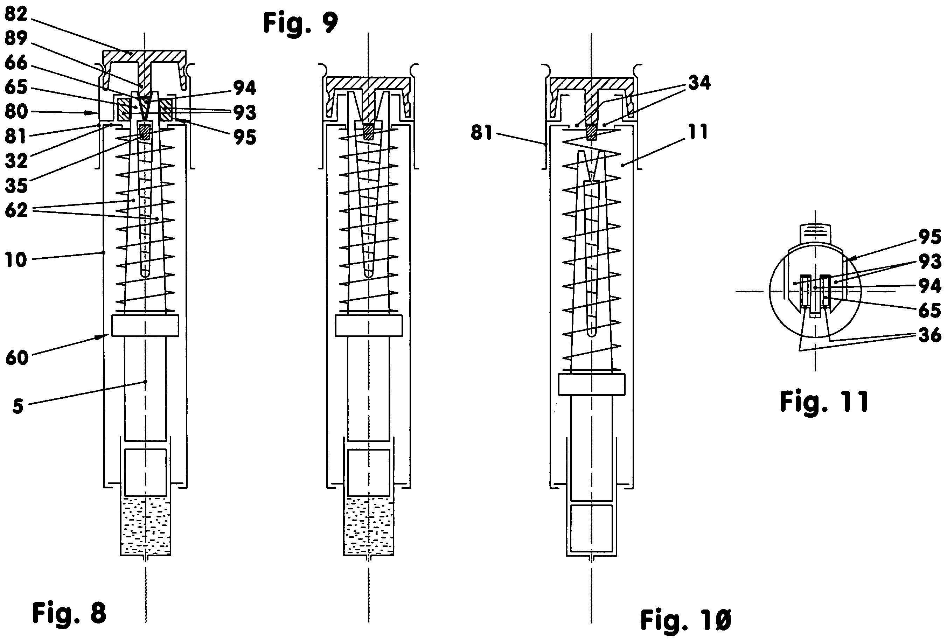

Mit den Figuren 8 bis 11 wird eine skizzierte Variante beschrieben, deren Zughaken (62) sich z.B. paarweise einander gegenüberliegen, d.h. die Abstützabschnitte (65) sind einander zugewandt. Die Zughaken (62) federn dabei aufeinander zu.

Nach Figur 8 hat das Gehäuse (10) dieser Variante im Boden (32) zwei z.B. rechteckige Ausnehmungen (34), die durch einen Gehäusesteg (35) getrennt sind. Der Gehäusesteg (35), auf dem die Abstützabschnitte (65) der Zughaken (62) - in Sperrstellung des Einweginjektors - aufliegen, ist Teil des Gehäusebodens (32). Um die Zughaken (62) sicher auf dem Gehäusesteg (35) zu halten, werden sie oberhalb des Gehäusebodens (32) bereichsweise umgriffen. Hierzu wird ein Sicherungselement (95) verwendet, vgl. auch Figur 11. Dieses Sicherungselement (95) ist im Wesentlichen ein gabelförmiges Bauteil mit drei Zinken und einem Griffteil, das im Auslösegehäuse (81) geführt ist. An den äußeren Zinken (93) liegen jeweils die Rückseiten der Abstützabschnitte (65) an. Zwischen den einander gegenüberliegenden Abstützabschnitten (65) befindet sich die mittlere Gabelzinke (94).

Im Auslösegehäuse (81) ist auch ein Auslöseelement (82) in Form eines Druckknopfes längsgeführt. Z.B. zentral am Auslöseelement ist ein Spreizstab (89) angeformt. Letzterer liegt nach Figur 8 auf dem mittleren Gabelzinken (94) des Sicherungselements (95) auf. Damit ist das Auslöseelement (82) bis zum Entfernen des Sicherungselements (95) blockiert.

Zum Auslösen des Einweginjektors wird zunächst das Sicherungselement (95) seitlich aus dem Auslösegehäuse (81) vollständig herausgezogen. Anschließend wird der Druckknopf (82) niedergedrückt, bis er am Gehäusesteg (35) aufliegt, vgl. Figur 9. Dabei wirken der Spreizstab (89) und die Keilfläche (66) wie ein Schiebekeilgetriebe zusammen. Die Zughaken (62) werden auseinandergespreizt, so dass sie ungehindert durch die Ausnehmungen (34) in den Gehäuseinnenraum (11) gleiten können, vgl. Figur 10, um dort als Teil des Kolbenbetätigungsstempels (60) auf den Kolben (111) zu wirken.

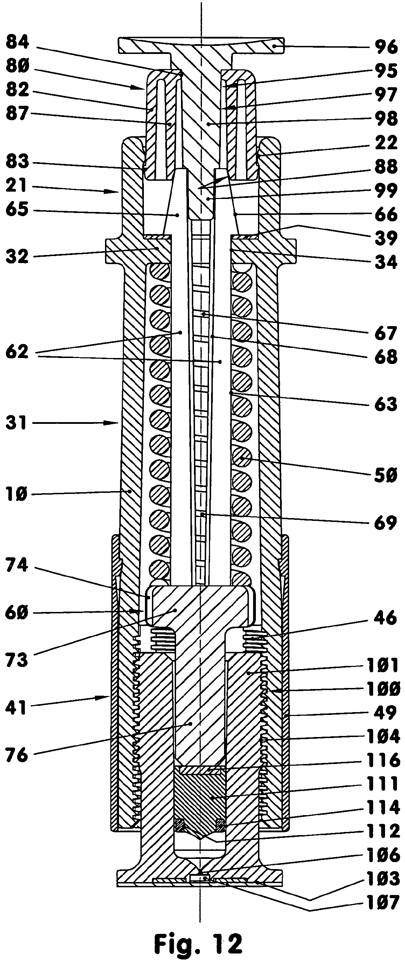

Die Figuren 12 bis 16 zeigen eine Ausführungsform des in den Figuren 4 bis 7 beschriebenen Prinzips. Hier ist das tragende Bauteil das Gehäuse (10). Es hat eine weitgehend rohrförmige Gestalt und ist in drei Funktionsbereiche (21, 31, 41) aufgeteilt. Nach der Figur 12 ist der obere Bereich der Auslösebereich (21). An ihn schließt sich der Mantelbereich (31) an. Zwischen beiden Bereichen ist ein Zwischenboden (32) angeordnet, der zudem geringfügig radial über den Mantelbereich (31) übersteht. Der Zwischenboden (32) hat eine zentrale Ausnehmung (34), deren Durchmesser sich z.B. nach unten hin geringfügig weitet.

Im Auslösebereich (21) des Gehäuses (10) befindet sich auf dem Zwischenboden (32) eine formsteife z.B. metallische Lochscheibe (39). Sie ist dort eingeklebt oder eingespritzt. Ggf. wird anstelle der Lochscheibe (39) auch eine keramische Panzerung verwendet. Die Lochscheibe (39) bzw. die Panzerung schützt den Zwischenboden (32) vor Eindrückungen und/oder anderen Verformungen. Sie verhindert auch ein Verkleben der sich dort ansonsten kontaktierenden Bauteile (32) und (65).

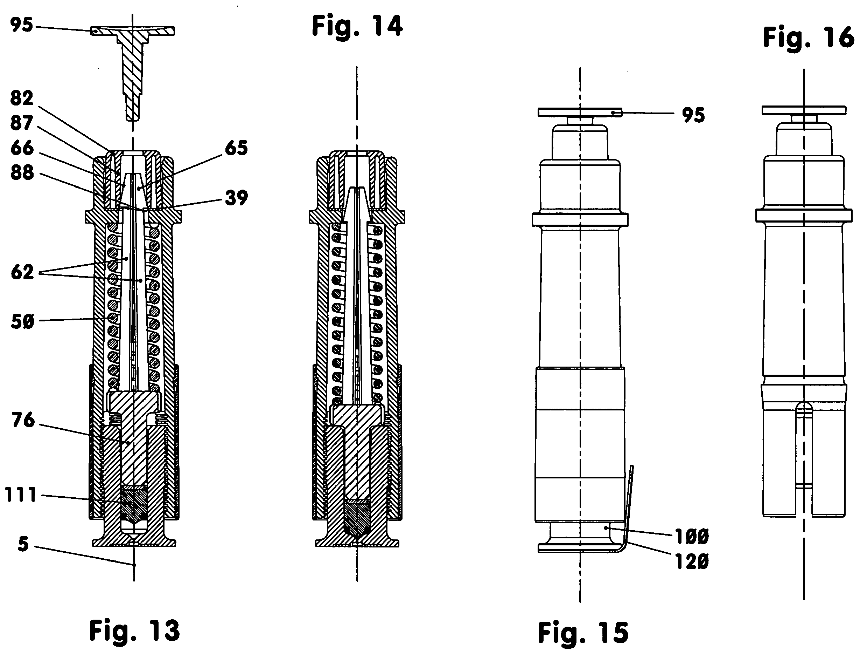

Unterhalb des Mantelabschnitts (31) befindet sich der Fixierbereich (41) zur Aufnahme der einbaubaren Zylinder-Kolben-Einheit (100). Der Fixierbereich (41) ist z.B. dreifach längsgeschlitzt, vgl. Figur 16. Die Innenwandung dieses Bereiches trägt z.B. ein Trapezgewinde (46). Nach den Figuren 12 und 15 ist der Fixierbereich (41) von einer am Gehäuse (10) verrasteten Stützhülse (49) umgeben.

In das Trapezgewinde (46) ist eine Zylinder-Kolben-Einheit (100), eingeschraubt. Letztere besteht aus einem Zylinder (101) und einem Kolben (111). Der Zylinder (101) ist ein z.B. dickwandiger Topf, dessen ggf. zylindrische Außenwandung zumindest bereichsweise ebenfalls ein Trapezgewinde (104) trägt.

In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist eine z.B. zylindrische Metallplatte (116) eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie mündet in einer zylindrischen Ausnehmung (107) der bodenseitigen, äußeren Stirnfläche (103) des Zylinders (101).

Zwischen dem Kolben (111) und dem Auslösebereich (21) ist der Federenergiespeicher (50) bzw. die Antriebseinheit des Einweginjektors angeordnet. Der Federenergiespeicher (50) ist eine Schraubendruckfeder, die auf einem Kolbenbetätigungsstempel (60) mit vier Zughaken (62) angeordnet ist. Mittels der Abstützabschnitte (65) der Zughaken (62) sitzt die Schraubendruckfeder (50) gespannt im Gehäuse (10). Letztere stützt sich zwischen der Innenseite des Zwischenbodens (32) und einer oberen Stirnseite des Kolbenbetätigungsstempels (60) ab.

Der Kolbenbetätigungsstempel (60) ist dabei in drei Bereiche aufgeteilt. Der untere Bereich ist der Kolbenschieber (76), der mittlere Bereich ist der das Federelement (50) abstützende Stempelteller (73) und der obere Bereich ist das Bündel aus z.B. vier Zughaken (62), vgl. auch Beschreibung zu den Figuren 1 bis 3.

Die Zughaken (62) haben im Mantelbereich (31) des Gehäuses (10) zumindest annähernd eine zylindrische Hüllfläche (63), d.h. ihre äußeren Wandungen haben die Krümmung eines Zylindermantelbereiches. Der Abstützabschnitt (65) hat als Hüllfläche einen Kegelstumpfmantel. Die Hüllfläche wird im Weiteren als Keilkontur (66) bezeichnet.

Die inneren Wandungen der Zughaken (62) sind Teile einer kegelstumpfmantelförmigen Hüllfläche (68). Diese Hüllfläche (68) umgibt den zwischen den Zughaken gelegenen kegelstumpfmantelförmigen Hohlraum (67). Hierbei vergrößern sich die Querschnitte des Hohlraums (67) je weiter sie sich vom Stempelteller (73) entfernen. Die radialen, zwischen benachbarten Zughaken (62) liegenden Schlitze (69) vergrößern sich gemäß Figur 12 nach oben hin auf die ca. doppelte Breite.

Abweichend zu Figur 1 hat der Stempelteller (73) nach Figur 12 z.B. zwei einander gegenüberliegende Nuten (74).

Die Zughaken (62) stecken mit ihren Abstützabschnitten (65) im Auslösebereich (21). Die Abstützabschnitte (65) liegen gesichert auf der Lochscheibe (39) auf.

Im Auslösebereich (21) sitzt eine Auslöseeinheit (80) als einteiliges Auslöseelement (82) in ihrer oberen Position. Das Auslöseelement (82) ist ein topfförmiger Körper, in dessen Inneren ein Auslöserohr (87) angeformt ist. Am unteren Ende der Außenkontur weist das Auslöseelement (82) einen umlaufenden, geringfügig überstehenden Rand (83) auf. Letzterer rastet hinter einem an der Innenwandung des Auslöseabschnitts (21) vorhandenen umlaufenden Wulst (22) ein.

Der Boden des Auslöseelements (82) hat eine kreisrunde Ausnehmung (84), in der ein Sicherungselement (95) eingesteckt ist. Das Auslöserohr (87) hat am unteren Ende im Bereich der Innenwandung eine kegelstumpfmantelförmige Keilkontur (88). Ihr Kegelwinkel beträgt im Ausführungsbeispiel zwischen 20 und 45 Winkelgraden. Die Keilkontur (88) liegt bei unbetätigtem und gesichertem Einweginjektor im oberen Bereich der Keilkontur (66) der Zughaken (62) an.

Das Sicherungselement (95) ist wie das Auslöseelement (82) ein rotationssymmetrisches Bauteil. Es besteht aus einem Teller (96) und einem Sperrstift (97). Der Sperrstift (97) hat einen Sperrbereich (99) und einen Stützbereich (98). Die beiden Bereiche (98, 99) sind kegelstumpfförmig. Sie haben z.B. beide den gleichen Kegelwinkel. Zumindest der Kegelwinkel des Sperrbereichs (99) entspricht dem Kegelwinkel des Hohlraumes (67). Der Stützbereich (98) stützt sich mit seiner ringförmigen Stirnfläche auf den oberen Stirnflächen der Zughaken (62) ab.

Die Figur 13 zeigt den Einweginjektor mit entferntem Sicherungselement (95) und betätigtem, also niedergedrücktem, Auslöseelement (82). Nach dem hier vertikalen Herausziehen des Sicherungselements (95) gleitet beim Niederdrücken des Auslöseelements (82) die Keilkontur (88) des Auslöserohrs (87) an den Keilkonturen (66) der Zughaken (62) entlang. Dabei werden die Zughaken (62) radial in Richtung Mittellinie (5) elastisch und/oder plastisch gebogen. Der Spaltraum zwischen den einzelnen Zughaken (62) wird dabei zumindest im Bereich der Abstützabschnitte (65) weitgehend verbraucht. Nun sind die maximalen Außendurchmesser der Abstützabschnitte (65) kleiner als der Durchmesser der Bohrung der Lochscheibe (39). Die Zughaken (62) können sich unter der Wirkung des Federelements (50) nach unten bewegen und den Kolben (111) mittels des Kolbenschiebers (76) verschieben, vgl. Figur 14.

Die Figur 15 zeigt den noch unbetätigten Einweginjektor in der handelsüblichen Aufmachung. Das Sicherungselement (95) ist eingesteckt und die untere Stirnseite der Zylinder-Kolben-Einheit (100) ist mit Hilfe einer abreißbaren Klebeversiegelung (120) steril verschlossen.

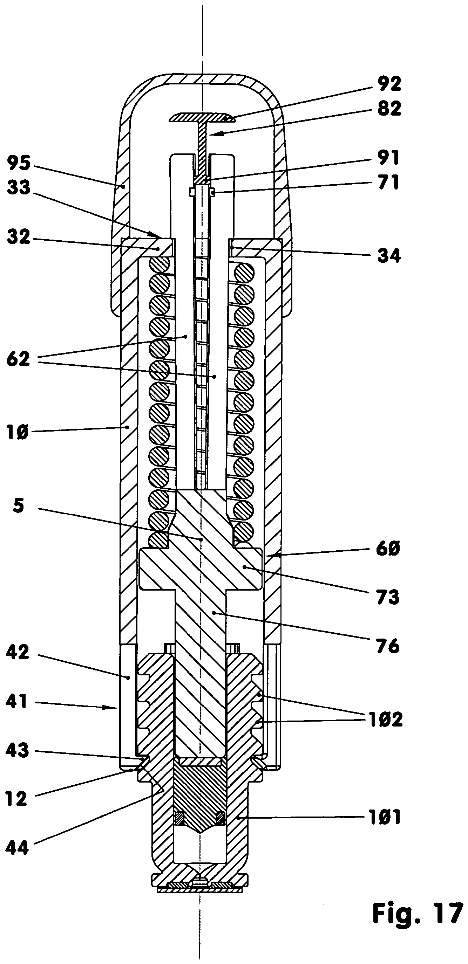

In den Figuren 17 bis 20 wird eine Einweginjektor-Variante dargestellt, deren Zughaken (62) zur Einweginjektormittellinie (5) hin elastisch federn. Das Gehäuse (10) ist im Wesentlichen ein glattes Rohr mit einem obenliegenden, ebenen Boden (32). Im Boden (32) ist zum Durchführen des Kolbenbetätigungsstempels (60) eine zentrale Bohrung (34) eingearbeitet.

Im unteren Bereich des Gehäuses (10) befindet sich der Fixierbereich (41) zur Aufnahme der einbaubaren Zylinder-Kolben-Einheit (100). Der Fixierbereich (41) umfasst z.B. sechs Federhaken (42), die jeweils in einer nach innen gerichteten Hakenspitze (43) enden. Die Hakenspitzen (43) haben zur unteren Gehäusestirnseite (12) hin eine sich über die gesamte Hakenstärke erstreckende Anfasung (44). Die Länge und die Federrate der Federhaken (42) ist so dimensioniert, dass die für die Funktion des Einweginjektors erforderlichen Einbauten (50, 100) ohne plastische Verformung der Federhaken (42) eingebaut werden können.

Einer dieser Einbauten ist die Zylinder-Kolben-Einheit (100), vgl. Figur 6. Sie besteht aus einem Zylinder (101) und einem Kolben (111), vgl. auch Figur 12. Der Zylinder (101) ist ein z.B. dickwandiger Topf, dessen ggf. zylindrische Außenwandung beispielsweise fünf umlaufende Rastrippen (102) trägt. Die Summe der Rastrippen (102) hat im Querschnitt z.B. ein Sägezahnprofil, wobei die Teilung zwischen den zahnartigen Rastrippen (102) äquidistant ist. Der maximale Durchmesser der Rastrippen (102) ist geringfügig kleiner als der Innendurchmesser des Gehäuses (10) im Fixierbereich (41). Der Durchmesser der zwischen benachbarten Rastrippen (102) liegenden Bereiche entspricht dem minimalen Durchmesser des Gehäuses (10) im Bereich der Hakenspitzen (43).

Im Gehäuse (10) sitzt zwischen dem Boden (32) und der Zylinder-Kolben-Einheit (100) der Kolbenbetätigungsstempel (60). Die unteren beiden Bereiche (73, 76) des Kolbenbetätigungsstempels (60) sind aus Figur 12 bekannt. Den oberen Bereich bilden z.B. vier Zughaken (62), die beispielsweise nicht bis zum Stempelteller (73) reichen. Zwischen dem Stempelteller (73) und dem Zughaken (62) liegt ein zylindrischer Abschnitt, der nach Figur 16 u.a. zur Führung des Federelements (50) dient. Er hat dazu z.B. vier kurze, radial abstehende Rippen, die die untere Windung des Federelements (50) fixieren.

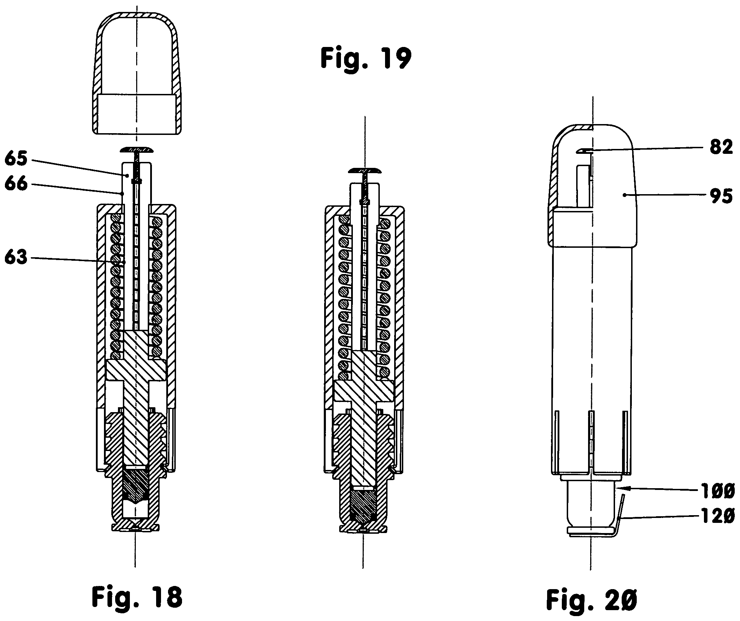

Jeder Zughaken (62) hat einen Abstützabschnitt (65). Das Zughakenbündel hat bei einem ausgelösten Einweginjektor eine zylindrische Hüllfläche (63), vgl. Figuren 18 und 19. Die Hüllfläche (66) kann auch im Bereich der Abstützabschnitte (65) zylindrisch sein.

Im oberen Bereich der Zughaken (62) befindet sich in jedem Zughaken (62) ein Teil einer Ringnut (71), deren Mittellinie mit der Mittellinie (5) des Einweginjektors deckungsgleich ist. In dieser Ringnut (71) sitzt nach den Figuren 18 und 19 eine z.B. zylindrische Spreizscheibe (91) eines Auslöseelements (82). Der Grund der jeweiligen Ringnutabschnitte liegt federnd an der radialen Außenkontur der Spreizscheibe (91) an. Im unbelasteten Zustand federn die Zughaken (62) in Richtung der Mittellinie (5). Beispielsweise würden sie sich beim Weglassen des Auslöseelements (82) an ihren oberen Enden berühren.

Das in Figur 17 gezeigte Auslöseelement (82) hat als rotationssymmetrisches Bauteil eine Pilzform. An der Spreizscheibe (91) ist ein schmaler Steg angeordnet, der mit seinem oberen Ende an einer Auslösescheibe (92) angeformt ist.

Zum Schutz des Auslöseelements (82) ist auf dem Boden (32) des Gehäuses (10) ein Sicherungselement (95) in Form einer Kappe aufgesteckt.

Die Figur 20 zeigt diesen Einweginjektor in der Seitenansicht mit halbseitig geschnittener Kappe (95). Die Zylinder-Kolben-Einheit (100) ist mit einer abziehbaren Schutzfolie (120) verschlossen. Nach Figur 16 befindet sich bei dem gesperrten Einweginjektor das Auslöseelement (82) in einer oberen Position. Die Spreizscheibe (91) sitzt oberhalb der Ringnut (71). Die Zughaken (62) nehmen eine aufgespreizte Position ein, wobei die Abstützabschnitte (65) auf der Oberseite (33) des Bodens (32) aufliegen.

Wenn nach dem Abnehmen der Kappe (95) und dem Abziehen der Schutzfolie (120) das Auslöseelement (82) in den Kolbenbetätigungsstempel (60) gedrückt wird, rastet die Spteizscheibe (91) des Auslöseelements (82) in die Ringnut (71) ein. Die Zughaken (62) federn zurück, so dass der maximale Außendurchmesser der Hüllfläche (66) der Abstützabschnitte (65) kleiner ist als der Durchmesser der Bohrung (34), vgl. Figur 18. Das Federelement (50) treibt nun den Kolbenbetätigungsstempel (60) nach unten, vgl. Figur 19. Mit der Abgabe des Medikaments über die Zylinder-Kolben-Einheit (100) ist der Injektionsvorgang beendet.

Mit Ausnahme der Federelemente (50, 64) sind alle Teile des Einweginjektors aus Kunststoffen oder kunststoff- bzw. gummiähnlichen Werkstoffen gefertigt.

### Bezugszeichenliste:

- 1: Injektionslösung; Medikament
- 5: Mittellinie des Einweginjektors

- 10: Gehäuse, einteilig
- 11: Gehäuseinnenraum
- 12: Gehäusestirnseite, unten
- 21: Auslösebereich
- 22: Wulst

- 31: Mantelbereich
- 32: Boden, Zwischenboden
- 33: Außenfläche, Stirnfläche, oben
- 34: Öffnung, Bohrung, Ausnehmung
- 35: Steg, Gehäusesteg
- 36: Gehäusekante, Rand
- 37: Auflagefläche
- 39: Lochscheibe

- 41: Fixierbereich für die Zylinder-Kolben-Einheit
- 42: Federhaken
- 43: Hakenspitze
- 44: Anfasung

- 46: Trapezgewinde
- 49: Stützhülse

- 50: Federelement, Schraubendruckfeder, Federenergiespeicher
- 60: Kolbenbetätigungsstempel
- 61: Zugstab
- 62: Zughaken
- 63: Hüllfläche, unten
- 64: Schraubendruckfeder, symbolisch
- 65: Abstützabschnitt
- 66: Außenkontur, keilförmig; Keilfläche; Keilkontur; Hüllfläche, oben
- 67: Hohlraum zwischen den Zughaken
- 68: Hüllfläche des Hohlraums
- 69: Schlitze zwischen den Zughaken
- 71: Ringnut
- 73: Stempelteller
- 74: Nuten

- 76: Kolbenschieber

- 80: Auslöseeinheit
- 81: Auslösegehäuse, Druckknopf
- 82: Auslöseelement
- 83: Rand
- 84: Ausnehmung für Sicherungselement
- 85: Hülse
- 86: Rechteckrohr
- 87: Auslöserohr
- 88: Keilflächen; Bereiche, abgeschrägt, Keilkontur
- 89: Spreizstab

- 91: Spreizscheibe
- 92: Auslösescheibe
- 93: äußere Zinken von (95)
- 94: Mittelzinke von (95
- 95: Sicherungselement, Kappe, Gabel
- 96: Teller
- 97: Sperrstift
- 98: Stützbereich
- 99: Sperrbereich

- 100: Zylinder-Kolben-Einheit
- 101: Zylinder
- 102: Rastrippen, außen
- 103: Stirnfläche
- 104: Trapezgewinde
- 106: Bohrung, Düse
- 107: Ausnehmung in der Stirnfläche

- 111: Kolben
- 112: Ringnut
- 114: Dichtring, Dichtung
- 116: Metallplatte

- 120: Schutzfolie, Klebeversiegelung

## Patentansprüche

1. Nadelfreier Einweginjektor mit einem Gehäuse (10), in dem oder an dem - jeweils zumindest bereichsweise - mindestens ein mechanischer Federenergiespeicher (50) angeordnet ist, mindestens eine - zumindest zeitweise wirkstoffbefüllbare - Zylinder-Kolben-Einheit (100) befestigt angeordnet ist, mindestens ein Kolbenbetätigungsstempel (60) angeordnet ist und mindestens eine Auslöseeinheit (80) angeordnet ist, wobei der Federenergiespeicher (50) mindestens ein vorgespanntes Federelement umfasst, wobei zumindest ein Teil des Kolbenbetätigungsstempels (60) zwischen dem Federenergiespeicher (50) und dem Kolben (111) der Zylinder-Kolben-Einheit (100) positioniert ist und wobei der federbelastete Kolbenbetätigungsstempel (60) mindestens einen zumindest bereichsweise querbeweglichen Zugstab (61) aufweist, der mittels eines Abstützabschnitts (65) den gespannten Federenergiespeicher (50) an mindestens einer Auflagefläche (37) des Gehäuses (10) abstützt, **dadurch gekennzeichnet,**
- **dass** in der Auslöseeinheit (80) mindestens ein Auslöseelement (82) quer zur Mittellinie (5) des Gehäuses (10) längsverschieblich gelagert ist, das bei einer Betätigung ein Wegbewegen des Abstützabschnitts (65) von der Auflagefläche (37) bewirkt oder freigibt.

2. Einweginjektor gemäß Anspruch 1, **dadurch gekennzeichnet, dass** der Zugstab (61) zusammen mit dem Abstützabschnitt (65) einen Zughaken (62) bildet, der in einer Sperrstellung die Gehäusekante (36) abstützend übergreift.

3. Einweginjektor gemäß Anspruch 2, **dadurch gekennzeichnet, dass** am Gehäuse (10) oder an der Auslöseeinheit (80) ein Sicherungselement (95) angeordnet ist, das den oder die Zughaken (62) in einer Sperrstellung sichert.

4. Einweginjektor gemäß Anspruch 1, **dadurch gekennzeichnet, dass** die Auslöseeinheit (80) mit dem Kolbenbetätigungsstempel (60) über ein Schiebekeilgetriebe (66, 88) mechanisch verbunden ist.

5. Einweginjektor gemäß Anspruch 2, **dadurch gekennzeichnet, dass** am Kolbenbetätigungsstempel (60) mindestens ein Bündel aus zwei oder mehr Zughaken (62) angeordnet ist.

6. Einweginjektor gemäß Anspruch 2, **dadurch gekennzeichnet, dass** der einzelne Zughaken (62) eine Länge hat, die größer als die halbe Länge des Kolbenbetätigungsstempels (60) ist.

7. Einweginjektor gemäß Anspruch 2, **dadurch gekennzeichnet, dass** bei einem Kolbenbetätigungsstempel (60) mit mindestens zwei Zughaken (62), die Schwerpunkte der Abstützabschnitte (65) der Zughaken (62) aufgrund ihrer Fertigung im unverformten Zustand weiter auseinander liegen als bei einem im Einweginjektor eingebauten, nicht ausgelösten Kölbenbetätigungsstempel (60).

## Claims

1. A needleless disposable injector having a housing (10), in which or on which - in each case at least in some regions - at least one mechanical spring energy reservoir (50) is disposed, at least one cylinder-piston unit (100) is mounted and disposed - which can at least occasionally be filled with active substance -, at least one piston-actuating ram (60) is disposed and at least one trigger unit (80) is disposed, wherein the spring energy reservoir (50) comprises at least one pre-tensioned spring element, wherein at least a portion of the piston-actuating ram (60) is positioned between the spring energy reservoir (50) and the piston (111) of the cylinder-piston unit (100), and wherein the spring-loaded piston-actuating ram (60) comprises at least one tension bar (61) which is transversely movable in at least some regions, which supports the tensioned spring energy reservoir (50) on at least one bearing surface (37) of the housing (10) by means of a support section (65), **characterized in that**
- in the trigger unit (80), at least one trigger element (82) is mounted transversely to the centre line (5) of the housing (10) in a longitudinally displaceable manner such that when actuated, the support section (65) is moved away from or is released from the bearing surface (37) .

2. The disposable injector as claimed in claim 1, **characterized in that** the tension bar (61) together with the support section (65) form a retaining hook (62) which, in a locked position, engages over the edge of the housing (36).

3. The disposable injector as claimed in claim 2, **characterized in that** a securing element (95) is disposed on the housing (10) or on the trigger unit (80), the securing element (95) securing the retaining hook or hooks (62) in a locked position.

4. The disposable injector as claimed in claim 1, **characterized in that** the trigger unit (80) is in mechanical communication with the piston-actuating ram (60) via a slide wedge drive (66, 88).

5. The disposable injector as claimed in claim 2, **characterized in that** at least one bundle of two or more retaining hooks (62) is disposed on the piston-actuating ram (60).

6. The disposable injector as claimed in claim 2, **characterized in that** the individual retaining hooks (62) have a length which is greater than half the length of the piston-actuating ram (60).

7. The disposable injector as claimed in claim 2, **characterized in that** when a piston-actuating ram (60) has at least two retaining hooks (62), because they are produced in the non-deformed state, the centres of gravity of the bearing sections (65) of the retaining hooks (62) are further apart than in the case of a piston-actuating ram (60) which has not been triggered installed in a disposable injector.

## Revendications

1. Injecteur sans aiguille à usage unique composé d'un boîtier (10) dans lequel ou sur lequel est disposé, au moins par endroits, au moins un accumulateur d'énergie mécanique à ressort (50), dans lequel ou sur lequel est fixée au moins une unité cylindre-piston (100) remplissable au moins temporairement d'une substance active, dans lequel ou sur lequel est disposé au moins un poinçon d'actionnement de piston (60) et au moins une unité de déclenchement (80), l'accumulateur d'énergie à ressort (50) comprenant au moins un élément à ressort précontraint, au moins une partie du poinçon d'actionnement de piston (60) étant positionné entre l'accumulateur d'énergie à ressort (50) et le piston (111) de l'unité piston-cylindre (100) et le poinçon d'actionnement de piston (60) chargé par ressort possédant une tige de traction (61) mobile transversalement au moins par endroits qui soutient l'accumulateur d'énergie à ressort précontraint au moyen d'une zone d'appui (65) sur au moins une surface de support (37) du boîtier (10), **caractérisé en ce**
- **qu'**au moins un élément de déclenchement (82) est placé dans l'unité de déclenchement (80), perpendiculairement à la ligne médiane du boîtier (10) de manière déplaçable longitudinalement, qui, lorsqu'il est actionné, provoque ou déclenche un éloignement de la zone d'appui (65) par rapport à la surface de support (37).

2. Injecteur à usage unique selon la revendication 1, **caractérisé en ce que** la tige de traction (61) et la partie de soutien (65) forment un crochet de traction (62) qui, dans une position de blocage, recouvre le bord du boîtier (36) en y prenant appui.

3. Injecteur à usage unique selon la revendication 2, **caractérisé en ce qu'**un élément de sécurité (95) qui fixe le ou les crochets de traction (62) dans une position de blocage, est disposé sur le boîtier (10) ou sur l'unité de déclenchement (80).

4. Injecteur à usage unique selon la revendication 1, **caractérisé en ce que** l'unité de déclenchement (80) est reliée mécaniquement au poinçon d'actionnement de piston à l'aide d'un engrenage à clavette coulissante (66, 88).

5. Injecteur à usage unique selon la revendication 2, **caractérisé en ce qu'**au moins un ensemble de deux ou plus de deux crochets de traction (62) est placé sur le poinçon d'actionnement de piston (60).

6. Injecteur à usage unique selon la revendication 2, **caractérisé en ce que** chaque crochet de traction (62) a une longueur supérieure à la moitié de la longueur du poinçon d'actionnement de piston (60).

7. Injecteur à usage unique selon la revendication 2, **caractérisé en ce que** dans le cas d'un poinçon d'actionnement de piston (60) équipé d'au moins deux crochets de traction (62) les centres de gravité des parties de soutien (65) des crochets de traction (62), dû à la fabrication, à l'état non déformé se trouvent plus éloignés l'un par rapport à l'autre que les centres de gravité d'un poinçon d'actionnement de piston (60) non déclenché intégré dans un injecteur à usage unique.
